# FASCICULE DE BREVET EUROPEEN

(11) **EP 3 393 408 B1**
(45) Date de publication et mention de la délivrance du brevet: **23.04.2025**
(21) Numéro de dépôt: 16831501.8
(22) Date de dépôt: 21.12.2016
(51) Int. Cl.: A61F 5/01, A61F 5/02, D03D 15/56, D02G 3/32, D03D 19/00

(54) **DISPOSITIF MEDICAL DESTINE A SOUTENIR AU MOINS UNE ZONE DU CORPS**
MEDIZINISCHE VORRICHTUNG ZUR UNTERSTÜTZUNG VON MINDESTENS EINES BEREICHS DES KÖRPERS
MEDICAL DEVICE FOR SUPPORTING AT LEAST ONE REGION OF THE BODY

(30) Priorité: 21.12.2015 FR 1562883
(43) Date de publication de la demande: 31.10.2018
(73) Titulaire: Thuasne, 92300 Levallois Perret (FR)
(72) Inventeur: BREUIL, Laurent, 42240 Unieux (FR); DE MONCUIT, Henri, Bakersfield, California 93309 (US)
(74) Mandataire: Cabinet Beau de Loménie
(86) Numéro de dépôt international: PCT/FR2016/053610
(87) Numéro de publication internationale: WO 2017/109410

(56) Documents cités:
- FR-A1- 2 156 436
- FR-A1- 2 934 151
- US-A- 3 920 054
- US-A- 4 207 885
- US-A1- 2014 303 537

## Description

La présente invention concerne le domaine technique des dispositifs médicaux destinés à soutenir au moins une zone du corps comprenant au moins un panneau textile élastique dans la direction longitudinale apte à être posé sur ladite zone du corps par l'intermédiaire d'un dispositif mécanique de mise en tension dudit panneau textile démultipliant la force manuelle de mise en tension du poseur dudit au moins un panneau textile.

### Arrière-plan de l'invention

Les dispositifs médicaux destinés à soutenir au moins une portion du corps, telles que les orthèses de genoux ou de cheville ou les ceintures lombaires, ont besoin d'être bien ajustés pour être efficaces. L'action de ces dispositifs de maintien est liée à une bonne contention de ladite zone du corps ciblée par le prescripteur. Par exemple une genouillère rotulienne est efficace si elle maintient correctement la rotule ce qui implique que la genouillère soit bien ajustée au niveau du mollet et de la cuisse. De nombreux patients, peu familiers des dispositifs médicaux de maintien, les adaptent mal à leurs pathologies, soit par ce que leurs morphologies ne s'y prêtent pas, soit et surtout parce qu'ils ne comprennent pas comment utiliser l'ensemble des éléments du dispositif médical de maintien.

US 4 207 885 A divulgue un bandage élastique de compression destiné à soutenir au moins une zone du corps tel que le genoux, le bandage ayant une direction longitudinale et une direction transversale agencé en sorte d'encercler tout ou partie de ladite zone du corps à soutenir, ladite bande tissée ayant un sens chaîne et un sens trame, et comprenant des fils de trame, des premiers fils de chaîne, des seconds fils de chaîne, et des troisièmes fils de chaîne, lesdits premiers fils de chaîne et lesdits seconds fils de chaîne sont tissés selon une armure pas-de-gaze, les seconds fils de chaîne formant les fils de tour, les sens chaînes et trames des bandes tissées sont parallèles entre-eux; les différents fils sont en coton et ledit panneau textile est rendu élastique dans la direction longitudinale de part l'armature pas-de-gaze.

En outre, ces dispositifs médicaux de maintien sont souvent très fastidieux à fermer alors que le patient peut avoir des problèmes de compréhension et même d'observance. En effet, un dispositif médical compliqué à mettre œuvre ne sera pas utilisé et la pathologie ne sera pas traitée.

Les dispositifs médicaux de maintien d'au moins une zone du corps, telle une ceinture de maintien lombaire, comprennent habituellement au moins un panneau textile élastique, c'est-à-dire apte à s'allonger dans au moins une direction sous l'effet d'une contrainte exercée uniquement manuellement par le patient et ayant une récupération élastique au moins supérieure ou égale à 90% de sa longueur initiale. Le panneau textile élastique utilisé est généralement un tissu comprenant en trame des fils élastiques.

Les contraintes imposées par certains systèmes de normalisation, en particulier par la sécurité sociale française, prévoient que les panneaux textiles des dispositifs médicaux de maintien doivent s'allonger d'au moins 30% sous l'effet d'une force minimum dont la valeur est déterminée selon le type de dispositif. Ainsi, il est requis que les ceintures de soutien lombaires s'allongent d'au moins 30% sous l'effet d'une force supérieure ou égale à 350 cN/cm et que les panneaux textiles des bande-ceintures de soutien lombaires s'allongement d'au moins 30% sous l'effet d'une force supérieure ou égale à 250 cN/cm, la récupération élastique devant être dans les deux cas supérieure ou égale à 90% de la longueur initiale de la dimension du panneau qui a été étirée. Il est donc nécessaire d'appliquer une force qui est supérieure ou égale à 350 cN/cm ou à 250 cN/cm respectivement pour les ceintures de soutien lombaire et les panneaux textiles des bande-ceintures de soutien lombaire pour obtenir un allongement de 30%. De tels dispositifs ont pour avantage qu'ils se plaquent parfaitement sur la zone du corps à soutenir. Cependant, ces dispositifs comprenant un panneau textile élastique s'allongeant sous une contrainte manuelle seule ne procurent pas une sensation de maintien et une sensation de serrage aussi importante que celles apportées par un textile inélastique, dit « rigide », qui est appliqué sur la zone à soutenir à l'aide d'un dispositif de serrage mécanique démultipliant la force de pose manuelle du patient.

De plus, il n'est pas possible de combiner un dispositif mécanique démultipliant la force de pose manuelle avec un panneau textile élastique car la course nécessaire au dispositif mécanique pour obtenir un serrage aussi important que celui obtenu à l'aide d'un panneau textile inélastique serait trop grande. Le serrage obtenu ne serait donc pas efficace. En outre, le dispositif mécanique de serrage démultipliant la force manuelle de pose serait trop complexe et encombrant pour pouvoir être rapporté sur un dispositif médical destiné à être porté par un utilisateur. Enfin, le temps de serrage pour obtenir la force de compression requise pour soutenir ladite au moins une zone du corps serait plus long que le temps de serrage nécessaire dans le cas d'un panneau textile inélastique.

Il existe ainsi un besoin pour un dispositif médical comprenant au moins un panneau textile ayant un comportement élastique dans au moins une direction, notamment apte à s'allonger d'au moins 30% de sa longueur initiale dans ladite direction avec une récupération élastique au moins supérieure ou égale à 90% de la longueur initiale qui a été étirée, et apte à être posé par l'intermédiaire d'un dispositif de serrage mécanique démultipliant la force de pose manuelle sans s'allonger, ou seulement de l'ordre de quelques pourcents, afin d'obtenir une sensation de serrage et donc de maintien équivalente à celles procurées par les textiles inélastiques.

### Objet et résumé de l'invention

L'invention est exposée dans le jeu de revendications annexé.

La présente invention a pour objet un dispositif médical destiné à soutenir au moins une zone du corps, notamment orthèse de genoux ou de cheville, ceinture de maintien ou bande de maintien, comprenant au moins un panneau textile ayant une direction longitudinale (L) et une direction transversale (T) agencé en sorte d'encercler tout ou partie de ladite zone du corps à soutenir. Avantageusement, ledit au moins un panneau textile comprend une bande textile tissée, ou plusieurs bandes textiles tissées superposées au moins partiellement, ladite bande tissée ou lesdites bandes tissées ayant, chacune, un sens chaîne (C) et un sens trame (t), et comprenant des fils de trame, des premiers fils de chaîne élastiques, des seconds fils de chaîne non élastiques, et des troisièmes fils de chaîne, lesdits premiers fils de chaîne et lesdits seconds fils de chaîne sont tissés selon une armure « pas de gaze », les seconds fils de chaîne formant les fils de tour. En outre, ledit au moins un panneau textile est élastique dans la direction longitudinale (L) en ce qu'il présente, dans cette direction (L) pour obtenir un allongement égal à 30 % une force qui est supérieure ou égale à 500 cN/cm, en particulier mesuré selon la norme NF EN 14704-1 de juin 2005.

Il a été trouvé que la combinaison de premiers fils de chaîne élastiques et de seconds fils de chaîne non élastiques selon une technique dite « pas de gaze » dans un tissu, en exerçant une traction importante sur lesdits premiers fils de chaîne élastiques en sorte de prendre de leur réserve d'élasticité, permet d'obtenir une bande tissée se comportant comme un textile rigide à la pose sous l'effet d'une force de traction manuelle, mais également rigide quand bien même ladite force de traction manuelle est démultipliée par un dispositif de serrage démultiplicateur de ladite force. L'expression « apte à » ou « agencé en sorte/pour » dans le présent texte est équivalente aux expressions suivantes, considérées indépendamment les unes des autres : « destiné à », « configuré pour, « adapté à autoriser » et « adapter à se conformer à ».

Le panneau textile comprenant une ou plusieurs bande(s) tissée(s) présente en outre un comportement élastique sous l'effet d'une force de traction très importante qui ne peut être atteinte manuellement, ni même à l'aide d'un dispositif de serrage démultipliant ladite force afin d'éviter une course de serrage trop importante lors de la pose dudit dispositif.

Il est donc nécessaire d'appliquer une force très importante dans la direction longitudinale (L) du panneau textile selon l'invention pour obtenir un allongement supérieur ou égal à 30%, en particulier une force supérieure ou égale à 500 cN/cm, de préférence une force supérieure ou égale à 700 cN/cm, encore de préférence supérieure ou égale à 1000 cN/cm, encore de préférence supérieure ou égale à 1000 cN/cm et inférieure ou égale à 2500 cN/cm.

De manière équivalente, la force nécessaire pour allonger le panneau textile selon l'invention dans la direction longitudinale (L) de 30%, est supérieure ou égale à 500 cN/cm, de préférence supérieure ou égale à 700 cN/cm, encore de préférence supérieure ou égale à 1000 cN/cm, encore de préférence supérieure ou égale à 1000 cN/cm et inférieure ou égale à 2500 cN/cm.

Le panneau textile selon l'invention bien qu'élastique sous une très forte contrainte procure au patient une très bonne sensation de soutien améliorant son confort et par la même l'observance de la pose du dispositif médical selon l'invention.

Les premiers fils de chaîne élastiques ne tournent pas autour d'un autre fil mais passent au-dessus ou en-dessous des fils de trame tel que cela est le cas de façon classique pour un fil de chaîne dans un tissu. Les premiers fils de chaîne élastiques ont ainsi une disposition parallèle au sens chaîne (C) de la bande tissée selon l'invention.

De préférence, ledit panneau textile, et notamment la ou lesdites bande(s) tissée(s), présente(nt) dans la direction longitudinale (L), ou le sens chaîne (C), un allongement supérieur ou égal à 30 % sous l'effet d'une force supérieure ou égale à 700 cN/cm, de préférence supérieure ou égale à 1000 cN/cm, encore de préférence sous l'effet d'une force supérieure ou égale à 1000 cN/cm et inférieure ou égale à 2500 cN/cm, ou de manière similaire, la force nécessaire pour allonger ledit panneau textile, et notamment la ou lesdites bande(s) tissée(s), dans la direction longitudinale (L), ou le sens chaîne (C), de 30%, est supérieure ou égale à 700 cN/cm, de préférence supérieure ou égale à 1000 cN/cm, encore de préférence supérieure ou égale à 1000 cN/cm et inférieure ou égale à 2500 cN/cm. En particulier, lesdits allongements et forces sont mesurés selon la norme NF EN 14704-1 de juin 2005.

Un panneau textile peut comprendre plusieurs bandes tissées superposées en sorte que les bords longitudinaux des bandes tissées soient parallèles entre-eux ou se croisent selon un angle déterminé. En particulier, les bandes tissées peuvent être superposées en sorte que les sens chaînes et trames (de préférence les sens chaînes) desdites bandes soient parallèles entre-eux ou en sorte que le sens chaîne ou trame (de préférence le sens chaîne) d'une bande tissée donnée soit disposée selon un angle alpha supérieur à 0° et inférieur à 90° par rapport au sens chaîne ou trame (de préférence le sens chaîne) d'une autre bande tissée, de préférence l'angle alpha est supérieur ou égal à 30° et inférieur ou égal à 60°. L'angle alpha peut être par exemple de l'ordre de 45°.

De préférence, les bords transversaux des bandes tissées superposées sont alignés.

La force nécessaire pour obtenir un allongement d'au moins 30% d'un panneau textile dans la direction longitudinale (L) est égale à la somme des forces appliquées individuellement à chaque bande tissée entrant dans la composition dudit panneau pour que chacune des bandes tissées s'allonge d'au moins 30% dans le sens chaîne (C).

Dans un mode de réalisation, ladite au moins une bande tissée est élastique dans le sens chaîne (C) en ce qu'elle présente un allongement supérieur ou égal à 30 % sous l'effet d'une force supérieure ou égale à 500 cN/cm, de préférence supérieure ou égale à 700 cN/cm, encore de préférence supérieure ou égale à 1000 cN/cm et inférieure ou égale 2500 cN/cm, en particulier mesuré selon la norme NF EN 14704-1 de juin 2005 ; ou de manière similaire, ladite au moins une bande tissée est élastique dans le sens chaîne (C) en ce qu'elle présente à un allongement de 30% une force appliquée qui est supérieure ou égale à 500 cN/cm, de préférence supérieure ou égale à 700 cN/cm, encore de préférence supérieure ou égale à 1000 cN/cm et inférieure ou égale 2500 cN/cm

La norme NF EN 14704-1 datant de juin 2005 est incorporée par renvoi dans le présent texte. Cette norme a pour titre « Détermination de l'élasticité des étoffes, Partie 1 : Essais sur bande ». Le principe est qu'une éprouvette d'étoffe de dimension spécifiée est étirée à vitesse constante jusqu'à soit une force spécifiée, soit un allongement spécifié pour un nombre convenu de cycles, et son élasticité est déterminée en mesurant plusieurs caractéristiques. L'appareil à vitesse constante d'allongement est défini au point 6 de ladite norme est doit notamment pouvoir fonctionner à des vitesses constantes d'allongement, en particulier à une vitesse d'allongement de 500 mm par minute. L'éprouvette d'étoffe testée peut être en bande (méthode A) ou en boucle (méthode B), de préférence en bande (méthode A). La force en cN/cm obtenue pour avoir un allongement supérieur ou égal à 30%, en particulier de l'ordre de 30%, est calculée en établissant la moyenne des forces mesurées à 30% d'allongement (ou plus) sur 5 cycles de traction. Selon une variante, le panneau textile comprend une seule bande tissée.

Selon une autre variante, le panneau textile comprend deux ou trois bandes tissées superposées.

La technique dite « pas-de-gaze » est habituellement utilisée dans le domaine des articles du prêt-à-porter ou décoratifs, par exemple pour les rideaux, tel que dans le tissu décrit dans US 3 920 054. Dans ce document, un fil élastique et un fil non élastique de tour sont disposés dans la même dent du peigne lors du tissage. On observe sur les figures que le fil de tour non élastique effectue un tour autour du fil élastique pour trois insertions de fil de trame. Cette technique de tissage dite également « leno » ou encore « tour anglais » permet de réaliser des décors ajourés dans les tissus.

US 4 207 885 décrit la fabrication d'un bandage de compression dans lequel la technique de tissage « pas-de-gaze » est utilisée. Les fils 12A et 12B tissés selon la technique dite pas-de-gaze sont des fils multifilamentaires en polyamide texturés. Lors du tissage, les fils de chaîne tissés selon la technique pas-de-gaze et les autres fils de chaîne sont mis en tension et alimentés de façon classique au métier à tisser, les fils de chaîne tissés deux-à-deux selon la technique pas-de-gaze étant davantage mis en tension que les autres fils de chaîne du fait du mouvement du fil de tour dans le sens chaîne. En sortie du métier à tisser, la bande tissée subit une étape de retrait thermique permettant de conférer à ladite bande une certaine extensibilité finale. Ce bandage est qualifié abusivement d'élastique bien qu'il ne comprenne pas de fil d'élastique.

Au sens de la présente invention, on comprend par panneau textile élastique ou bande tissée élastique dans un sens ou direction déterminé(e), que ce soit le sens chaîne et/ou le sens trame et/ou la direction longitudinale (L), ou fil élastique, notamment premier fil de chaîne élastique et/ou troisième fil de chaîne élastique, que ledit panneau ou ladite bande tissée a dans ladite direction longitudinale (L) ou dans ledit sens, ou ledit fil a, une récupération élastique au moins supérieure ou égale à 90%, en particulier mesurée selon la norme NF S97-115 datant de décembre 2011 combinée avec la norme NF EN 14704-1 datant de juin 2005.

Au sens de la présente invention, on comprend par panneau textile non élastique ou bande tissée non élastique dans un sens (chaîne ou trame) ou une direction (longitudinale ou transversale) déterminé(e), en particulier dans le sens trame (t) ou la direction transversale (T), ou fil non élastique, notamment second fil de chaîne non élastique et/ou troisième fil de chaîne non élastique, que ledit panneau ou ladite bande tissée a dans ladite direction transversale (T) ou ledit sens trame (t), ou ledit fil, a une récupération élastique inférieure à 90%, en particulier mesurée selon la norme NF S97-115 datant de décembre 2011 combinée avec la norme NF EN 14704-1 datant de juin 2005.

De préférence, le panneau textile et la bande tissée selon l'invention ne sont pas élastiques respectivement dans la direction transversale (T) et dans la direction trame (t).

Dans un mode de réalisation, le dispositif de maintien selon l'invention comprend une première extrémité et une seconde extrémité, la première extrémité comprend un premier organe d'attache et la seconde extrémité comprend un second organe d'attache, lesdits premiers et seconds organes d'attache étant aptes à coopérer ensemble pour leur fermeture et donc corrélativement la solidarisation dudit dispositif de maintien sur ladite au moins une zone du corps à soutenir.

Les premier et second organes d'attache peuvent être des organes d'attache du type à boucles et/ou à crochets, ou à aimants ou tout autre organe d'attache connu de l'homme du métier.

Avantageusement, le dispositif médical de maintien selon l'invention comprend un dispositif de serrage mécanique démultiplicateur de la force de pose manuelle dudit dispositif médical de maintien, notamment comprenant un dispositif de serrage par câble(s) et/ou un dispositif de serrage par poulie(s).

Le ou les premier(s) fil(s) de chaîne, est/sont un ou des fil(s) monofilamentaire(s) en élasthanne, c'est-à-dire à base de polyuréthane, par exemples tels que ceux commercialisés sous la marque Dorlastan^{®}, Glospan^{®}, Linel^{®}, Creora^{®} ou encore Lycra^{®}. Le ou les fil(s) élastique(s) peut/peuvent être également un ou des fil(s) monofilamentaire(s) en latex, c'est-à-dire à base de gomme naturelle ou synthétique, tel que par exemple l'élastodiène, ou dans tout autre matériau équivalent connu de l'état de la technique. Le ou lesdits fil(s) élastique(s) monofilamentaire(s) peut/peuvent également former l'âme élastique, laquelle âme est guipée ou recouverte d'un ou plusieurs fils de couverture, de préférence tel(s) que défini(s) ci-dessous en référence au(x) fil(s) filés de fibres et au(x) fil(s) multifilamentaires.

Le(s) fils de trame et/ou le(s) second(s) fil(s) de chaîne non élastique(s) et/ou le(s) troisième(s) fil(s) de chaîne est/sont choisi(s) dans le groupe constitué par un/des fil(s) multifilamentaire(s), un/des fil(s) monofilamentaire(s) et un/des fil(s) filés de fibres.

Le(s) troisième(s) fil(s) de chaîne peut/peuvent être élastique(s) ou non. Si le(s) troisième(s) fil(s) de chaîne est/sont élastique(s), il(s) se présente(nt) de préférence selon la même structure et/ou la même composition que le(s) premier(s) fil(s) de chaîne.

De préférence, le(s) troisième(s) fil(s) de chaîne n'est/ne sont pas élastique(s).

Le(s) fil(s) de trame et/ou le(s) premier(s) fil(s) de chaîne, le(s) second(s) fil(s) de chaîne et/ou le(s) troisième(s) fil(s) de chaîne, éventuellement le(s) fil(s) de couverture peut/peuvent être coloré(s) ou transparent(s) selon le rendu esthétique souhaité.

Le(s) fils de trame et/ou le ou les fil(s) de couverture du/des premier(s) fil(s) de chaîne et/ou le(s) second(s) fil(s) de chaîne et/ou le(s) troisième(s) fil(s) de chaîne (lorsqu'il(s) n'est/ne sont pas élastique(s)) est/sont dans un ou plusieurs matériaux choisi(s) dans le groupe comprenant : le polyamide 6, le polyamide 6-6, le polyamide 12, le polypropylène, le polyéthylène, le polyester tel que le polyéthylène téréphtalate, le coton, la viscose, le polyacrylique.

La bande tissée est dans un tissu ayant un nombre de fils de trame par centimètre (mesurée selon la direction chaîne (C)) inférieur ou égal à 25, encore de préférence inférieur ou égal à 15.

On entend par nombre de fils par centimètre, en chaîne ou en trame dans le panneau tissé selon l'invention, le nombre de fils sur le tissu au repos, en particulier après toutes éventuelles opérations de thermofixation appliquées à ladite bande tissée en sortie du métier à tisser, par centimètre dans la direction chaîne ou trame.

L'un au moins desdits premiers fils de chaîne est recouvert par au moins un premier fil de couverture.

Cette disposition permet d'ajuster la tension appliquée aux premiers fils de chaîne pour l'obtention d'un panneau textile rigide au sens de l'invention mais élastique sous une très forte contrainte.

De préférence, ledit premier fil de couverture forme un nombre de tours/m sur ledit au moins un premier fil de chaîne compris entre 720 tours/m et 1080 tours/m.

Selon un mode de réalisation, l'un au moins desdits premiers fils de chaîne est recouvert par un premier fil de couverture et un second fil de couverture.

De préférence, le second fil de couverture forme un nombre de tours/m sur ledit au moins un premier fil de chaîne compris entre 590 tours/m et 880 tours/m.

Dans une variante, l'un au moins desdits premiers fils de chaîne élastiques, éventuellement recouvert par au moins un premier fil de couverture (et éventuellement un second fil de couverture), présente un allongement à rupture inférieur ou égal à 150%, de préférence supérieur ou égal à 50%, encore de préférence supérieur ou égal à 75%.

Cet allongement à rupture peut être mesuré selon la norme NF ISO 2062 datant de 2010, s'appliquant notamment aux fils texturés synthétiques. Cet allongement à rupture peut être mesuré sur ledit au moins un fil de chaîne élastique avant qu'il ne soit tissé ou ourdie.

Dans le procédé permettant de fabriquer la bande tissée selon l'invention, une tension très importante est exercée sur ledit au moins un premier fil élastique en sorte de prendre de sa réserve d'élasticité. Ledit au moins un premier fil élastique a ainsi moins d'allongement dans la bande tissée que l'allongement qu'il présente avant d'avoir été mis en œuvre par tissage, c'est-à-dire sur sa bobine d'alimentation.

En effet, un fil élastique de l'état de la technique a un allongement à rupture généralement au minimum de l'ordre de 400% à 500% mesuré sur sa bobine d'alimentation avant toute mise en œuvre par tissage.

Les seconds fils de chaîne effectuent un tour pour une insertion d'un fil de trame.

De préférence, lesdits seconds fils de chaîne effectuent un tour pour une insertion de fil de trame sur au moins une portion de la longueur en chaîne de chaque bande tissée.

Cette disposition permet de bloquer les premiers fils de chaîne élastiques en sorte de prendre de leur réserve d'élasticité, c'est-à-dire diminuer leur allongement à rupture disponible. Dans l'état de la technique, le fil de tour effectue généralement un seul tour pour deux ou trois fils de trame insérés alors que dans la présente invention les seconds fils de chaîne non élastiques forment un tour pour une seule insertion de fil de trame afin de bloquer et contraindre les premiers fils de chaîne élastiques.

L'un au moins desdits fils de trame comprend un fil ayant un nombre de filaments supérieur ou égal à 1 et inférieur ou égal à 5, de préférence un nombre de filaments supérieur ou égal à 1 et inférieur ou égal à 3, chaque filament ayant un diamètre supérieur ou égal à 0,10 mm, notamment inférieur ou égal à 0,60 mm, en particulier inférieur ou égal à 0,45 mm.

Cette disposition favorise la rigidité de la bande tissée dans le sens trame mais également dans le sens chaîne.

Dans une variante, la bande tissée ou plusieurs bandes tissées comprend(nnent) une alternance de colonnes pleines et de colonnes ajourées, chacune desdites colonnes pleines et ajourées ayant une direction longitudinale (I) correspondant au sens chaîne (C) de ladite bande tissée, les colonnes pleines comprenant selon chacun de leurs bords longitudinaux au moins un premier fil de chaîne et un second fil de chaîne assemblés selon une armure pas de gaze.

De préférence, les colonnes pleines comprennent les premiers fils de chaînes élastiques, les seconds fils de chaîne non élastiques, les troisièmes fils de chaîne et les fils de trame tels que définis dans le présent texte.

De préférence, les colonnes pleines et les colonnes ajourées sont alternées une à une.

De préférence, les colonnes pleines ne sont pas ajourées par opposition aux colonnes ajourées.

Les colonnes ajourées comprennent ainsi des jours ayant au moins une dimension supérieure ou égale à 1 mm. Ces jours favorisent l'évacuation de la chaleur et de la transpiration du porteur.

Dans une variante, les colonnes ajourées sont constituées de fils de trame.

Dans une variante, le dispositif médical selon l'invention comprend un dispositif de serrage démultiplicateur de la force de pose manuelle dudit au moins un panneau textile, notamment un dispositif de serrage mécanique comprenant un dispositif de serrage par câble(s) et/ou un dispositif de serrage par poulie(s). Ce dispositif de serrage démultiplicateur permet de multiplier par deux, trois, voire plus, la force manuelle de pose dudit au moins un panneau textile.

La présente invention a pour objet, selon un second aspect, un procédé de fabrication d'un dispositif selon l'une quelconque des variantes de réalisation précédentes en référence à un premier aspect, comprenant les étapes suivantes :
- une première étape de tissage d'une bande textile tissée, ou de plusieurs bandes textiles tissées, chaque bande textile étant tissée avec des premiers fils de chaîne élastiques, des seconds fils de chaîne non élastiques, des troisièmes fils de chaîne, et des fils de trame, les premiers fils de chaîne et les seconds fils de chaîne étant tissés selon une armure pas de gaze en sorte que les seconds fils forment les fils de tour ;
- une étape d'application d'une tension (daN) sur l'un au moins desdits premiers fils de chaîne élastiques, de préférence sur l'ensemble des premiers fils de chaîne élastiques, en sorte d'allonger ledit au moins premier fil de chaîne élastique lors de ladite première étape ;
- une étape de formation d'un panneau textile ayant des directions longitudinale (L) et transversale (T) comprenant une bande textile tissée, ou plusieurs bandes textiles tissées superposées, ledit panneau textile présente dans la direction longitudinale (L) pour obtenir un allongement égal à 30%, une force qui est supérieure ou égale à 500 cN/cm, en particulier mesuré selon la norme NF EN 14704-1 de juin 2005.

Dans une sous-variante, ladite bande textile tissée ou lesdites bandes textiles tissées subit/subissent une étape d'apprêtage.

Dans un mode de réalisation préféré, ladite étape d'apprêtage de la bande textile tissée comprend les étapes suivantes :
- une première étape de post-traitement thermique consistant à plonger la bande tissée dans un bain d'eau chaude pendant au moins une seconde, de préférence pendant au moins 15 secondes, la température de l'eau dudit bain est de préférence supérieure ou égale à 60°C ; ou
- une première étape de post-traitement thermique consistant à plonger la bande tissée dans un bain d'eau à température ambiante (de préférence dont la température est supérieure ou égale à 10°C et inférieure ou égale à 30°C), ledit bain comprenant au moins un additif, ledit additif étant choisi de préférence en sorte d'attribuer de la rigidité (« main » à la bande tissée) ou de la souplesse ; et ensuite
- une seconde étape de post-traitement thermique consistant à passer la bande tissée dans une chambre de traitement comprenant de l'air chaud, ou de l'air humide, de préférence la température de l'air est supérieure ou égale à 60°C (et inférieure ou égale à 150°C), puis à calandrer ladite bande tissée par passage sur au moins un cylindre chauffé, notamment sur un ensemble de cylindres chauffés, afin d'obtenir son retrait dimensionnel.

Ledit au moins un additif peut être un polymère de silicone ou de polyuréthane. L'homme du métier sait quel(s) additif(s) choisir pour impartir de la rigidité ou de la souplesse à une bande textile tissée.

En outre, la surface externe du cylindre ou des cylindres lors du calandrage de la seconde étape de post-traitement thermique peut être métallique, et/ou éventuellement revêtue d'une enduction dans un polymère non adhérent, par exemple un polymère fluoré tel que le PTFE. L'homme du métier sait ajuster la température du ou desdits cylindres ainsi que sa/leurs vitesse(s) de rotation en sorte d'obtenir la relaxation de la bande textile et la rétractation dimensionnelle de cette dernière.

Cette étape de post-traitement permet de donner au tissu ses caractéristiques mécaniques finales, grâce à la rétractation des différents fils constituant le tissu. Les variantes de réalisation et définitions décrites ci-dessus en référence au premier aspect de l'invention s'appliquent également au procédé.

Dans une variante, l'étape d'application d'une tension (daN) consiste à appliquer une tension sur l'un au moins desdits ou lesdits premiers fils de chaîne élastiques à l'aide d'un ou plusieurs dispositif(s) choisi(s) dans le groupe comprenant les dispositifs suivants: l'un au moins desdits premiers fils de chaîne élastiques est recouvert par au moins un premier fil de couverture ayant un nombre de tours/mètre supérieur ou égal à 720 tours/m, et/ou une tension exercée sur l'un au moins desdits premiers fils de chaîne élastiques supérieure ou égale à 10 cN, et/ou un nombre de tours des seconds fils de chaîne égal à un pour une insertion d'un fil de trame.

La tension exercée sur un fil (mesurée en cN) peut être mesurée par exemple à l'aide d'un tensiomètre.

### Description détaillée des dessins

La présente invention sera mieux comprise à la lecture des exemples de réalisation suivants, cités à titre non limitatif, illustrés par les figures suivantes annexées à la présente, et dans lesquelles :
- la figure **1** est une représentation schématique d'un premier exemple de bande tissée selon l'invention ;
- la figure **2** est une représentation schématique de l'agencement des fils d'une colonne pleine entre deux colonnes ajourées de la bande tissée représentée à la figure **1** **;**
- la figure **3** est une représentation schématique vue selon sa face postérieure d'un premier exemple de dispositif médical selon l'invention comprenant deux panneaux textiles, chaque panneau textile comprenant deux bandes textiles tissées telle que la bande tissée représentée à la figure **1****,** ledit dispositif médical est dans cet exemple précis une ceinture de soutien lombaire.

### Description détaillée des exemples de réalisation

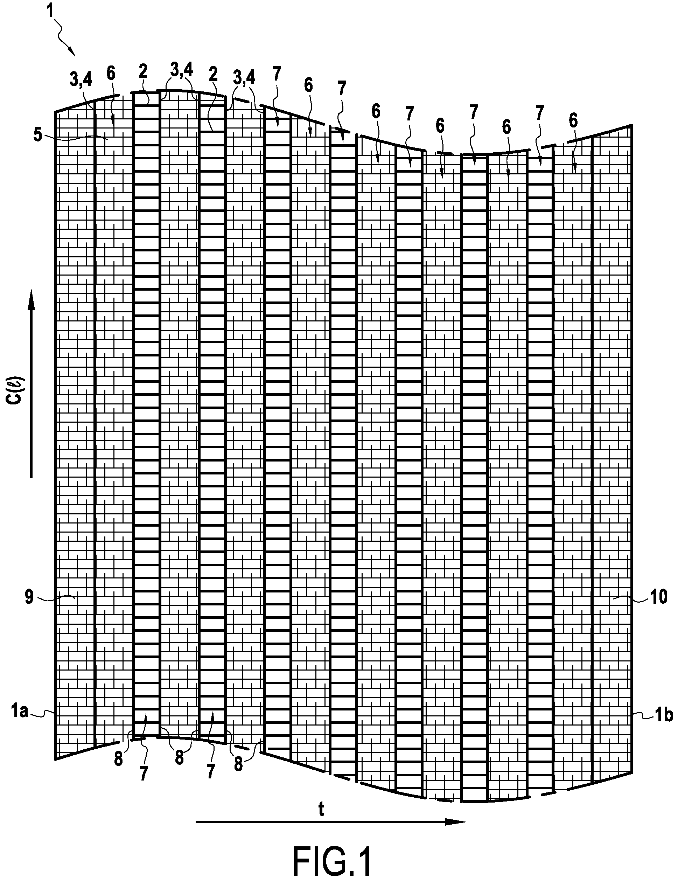

La bande textile tissée **1** selon l'invention représentée à la figure **1** a un sens chaîne **C** et un sens trame **t.** Ladite bande tissée **1** est un tissu comprenant des fils de trame **2,** des premiers fils de chaîne élastiques **3,** des seconds fils de chaîne non élastiques **4,** et des troisièmes fils de chaîne **5,** lesdits premiers fils de chaîne **3** et lesdits seconds fils de chaîne **4** sont tissés selon une armure « pas de gaze », les seconds fils de chaîne **4** formant les fils de tour. Ladite bande tissée **1** est élastique dans le sens chaîne **C** et présente dans le sens chaîne, dans cet exemple précis, un allongement supérieur ou égal à 30% sous l'effet d'une force supérieure ou égale à 700 cN/cm, en particulier mesuré selon la norme NF EN 14704-1 de juin 2005. Ainsi, pour obtenir un allongement de 30% dans la direction longitudinale **L,** il est requis d'appliquer audit panneau textile dans cette direction **L,** une force supérieure ou égale à 700 cN/cm.

La bande tissée **1** comprend une alternance de colonnes pleines **6** et de colonnes ajourées **7,** chacune desdites colonnes pleines **6** et ajourées **7** ayant une direction longitudinale **l** correspondant au sens chaîne **C** de ladite bande tissée **1.** De préférence, chacune des colonnes pleines **6** comprend sur chacun de ses bords longitudinaux **8** au moins un premier fil de chaîne élastique **3** et un second fil de chaîne non élastique **4** tissés selon une armure pas-de-gaze.

Les colonnes ajourées **7** comprennent des fils de trame **2** s'étendant dans la direction trame **t,** transversalement à la direction chaîne **C.**

La bande tissée **1** comprend selon chacun de ses bords longitudinaux **1a,1b** respectivement une lisière **9,10.**

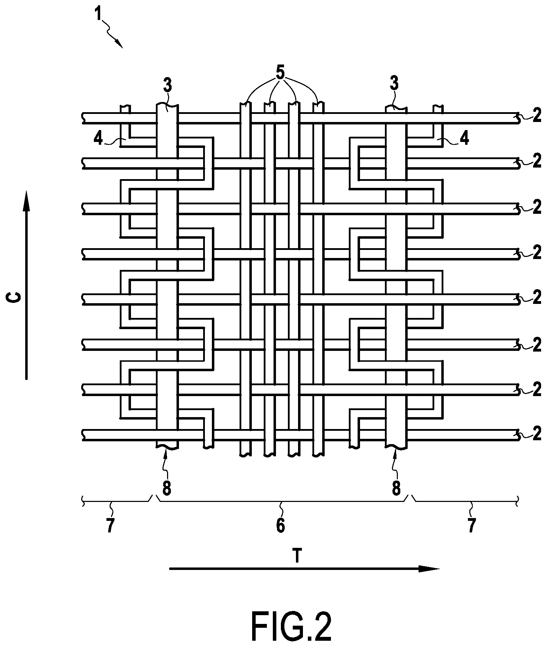

La figure **2** représente de façon schématique une colonne pleine **6** comprenant sur chacun de ses bords longitudinaux **8,** un premier fil de chaîne élastique **3** et un second fil de chaîne non élastique **4** tissés ensemble selon une armure pas de gaze. La colonne pleine **6** est disposée entre deux colonnes ajourées **7** constituées par les fils de trame **2.** La colonne pleine **6** comprend des troisièmes fils de chaîne **5** disposées entre les premiers **3** et seconds **4** fils de chaîne tissés selon une armure pas de gaze.

Tel que cela est visible à la figure **2****,** les seconds fils de chaîne **4** effectuent un tour autour des premiers fils de chaîne **3** pour une insertion d'un fil de trame **2.** L'un au moins desdits fils de trame **2** est un fil comprenant un nombre de filaments supérieur ou égal à 1 et inférieur ou égal à 5, de préférence comprenant un nombre de filaments supérieur ou égal à 1 et inférieur ou égal à 3, dans cet exemple précis égal à 3, chaque filament ayant un diamètre supérieur ou égal à 0,1 mm.

Dans cet exemple précis, ladite bande tissée **1** présente dans le sens chaîne **C** un allongement supérieur ou égal à 30% sous l'application d'une force supérieure ou égale à 700 cN/cm. Ainsi, pour obtenir un allongement de 30% dans la direction longitudinale **L,** il est requis d'appliquer à ladite bande **1** dans cette direction **L,** une force supérieure ou égale à 700 cN/cm.

De préférence, les troisièmes fils de chaîne **5** et les seconds fils de chaîne **4** ne sont pas élastiques, et sont par exemple des fils multifilamentaires ou des fils filés de fibres ayant un titre supérieur ou égal à 50 dtex et inférieur ou égal à 500 dtex, pour un nombre de filaments comprise entre 10 et 300.

Les premiers **3,** seconds **4** et troisième **5** fils de chaîne ainsi que les fils de trame **2** peuvent être transparent ou colorées. En outre, lesdits fils peuvent être en polyamide 6, polyamide 6-6, en polyester, en polyéthylène téréphtalate, en polypropylène, en coton, ou toute autre matière naturelle ou synthétique ou artificielle non élastique.

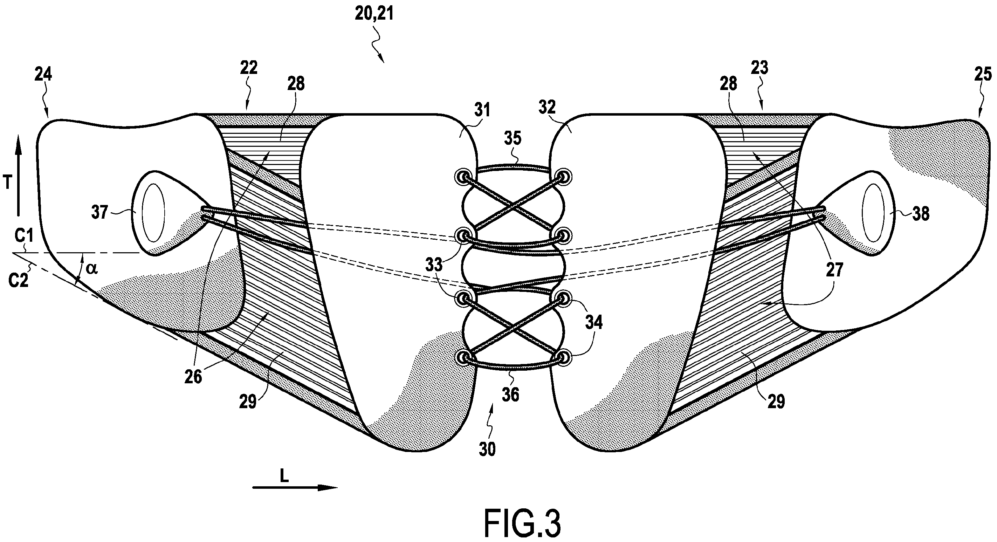

La figure **3** représente un dispositif médical **20** selon l'invention destiné à soutenir au moins une zone du corps. Dans cet exemple précis, il s'agit d'une ceinture de maintien lombaire **21.** Ledit dispositif médical **20** comprend une partie latérale gauche **22** et une partie latérale droite **23** destinées, en fonctionnement et donc au porté, à recouvrir respectivement le flanc gauche et au moins une portion dorsale postérieure gauche, et le flanc droit et au moins une portion dorsale postérieure droite du porteur.

Le dispositif médical **20** comprend une première extrémité **24** et une seconde extrémité **25** pourvues respectivement d'un premier organe d'attache et d'un second organe d'attache, lesdits premier et second organes d'attache étant aptes à coopérer pour permettre la fermeture de la ceinture de maintien lombaire **21** sur le tronc du porteur. Lesdits premier et second organes d'attache étant par exemple respectivement des crochets d'attache et un textile ayant des boucles d'attache.

Chaque partie latérale **22,23** du dispositif **20** comprend respectivement un panneau textile **26,27** selon l'invention. Chaque panneau textile **26,27** comprend respectivement deux bandes tissées **28, 29** correspondant chacune à la bande tissée **1** représentée à la figure **1****.** Les bandes tissées **28,29** sont superposées et agencées entre-elles en sorte que le sens chaîne **C1** de la bande tissée **28** forme un angle alpha de l'ordre de 45° avec le sens chaîne **C2** de la bande tissée **29.**

Chaque panneau textile, **28** et **29,** présente dans la direction longitudinale un allongement supérieur ou égal à 30% sous l'application d'une force supérieure ou égale à 700 cN/cm, notamment comprise entre 1400 cN/cm et 2000 cN/cm. Il est donc nécessaire d'appliquer une force comprise entre 1400 cN/cm et 2000 cN/cm dans la direction longitudinale desdits panneaux textiles **28,29** pour obtenir un allongement d'au moins 30% dans cette direction **L.**

Le dispositif médical **20** comprend en outre un dispositif de serrage mécanique démultiplicateur de la force de pose manuelle **30** disposé entre les parties latérales gauche **22** et droite **23.** Dans cet exemple précis, ce dispositif de serrage démultiplicateur **30** comprend des première **31** et seconde **32** portions latérales comprenant chacune plusieurs œillets **33,34,** en particulier quatre œillets, pour le passage de deux éléments longilignes **35,36,** tels des cordons. Les œillets **33** et les œillets **34** respectivement des première **31** et seconde **32** portions latérales sont en vis-à-vis en sorte de former de multiples renvois des éléments longilignes **35,36** démultipliant la force de pose. Deux organes de préhension **37,38,** notamment deux poignées, sont solidarisées via les éléments longilignes **35,36** audit dispositif de serrage mécanique **30** et sont actionnables par le poseur pour disposer la ceinture de maintien lombaire **21** sur son dos et serrer cette dernière en sorte de faire coopérer lesdits premier et second organes d'attache ensemble pour la fermeture dudit dispositif médical **20.**

En fonctionnement, le patient dispose la ceinture de soutien lombaire **21** sur son torse, actionne le dispositif de serrage démultiplicateur **30** en tirant sur les poignées **37,38** en sorte de plaquer et serrer la ceinture **21** sur son dos, puis fait coopérer les premier et second organes d'attache pour la solidarisation de la ceinture **21.** Les panneaux textiles **26,27,** bien qu'élastiques dans la direction longitudinale **L** sous une très forte contrainte, se comportent lors de la pose et de l'utilisation avec ledit dispositif de serrage démultiplicateur **30,** tels des panneaux textiles rigides. Ainsi, l'allongement obtenu même sous la force manuelle démultipliée desdits panneaux textiles **26,27** dans la direction longitudinale **L** est très faible, par exemple de l'ordre de quelques pourcents (2 à 5 %) sans une course de serrage importante.

## Revendications

1. Dispositif médical (20) destiné à soutenir au moins une zone du corps, notamment orthèse de genoux ou de cheville, ceinture de maintien (21) ou bande de maintien, comprenant au moins un panneau textile (26,27) ayant une direction longitudinale (L) et une direction transversale (T) agencé en sorte d'encercler tout ou partie de ladite zone du corps à soutenir, **caractérisé en ce que** :
- ledit panneau textile comprend plusieurs bandes textiles tissées superposées (1,28,29) au moins partiellement,
- lesdites bandes tissées (1,28,29) ayant chacune un sens chaîne (C) et un sens trame (t), et comprenant chacune des fils de trame (2), des premiers fils de chaîne élastiques (3) dont un ou des premier(s) fil(s) de chaine élastique(s) (3) est/sont un ou des fil(s) monofilamentaire(s), en élasthanne ou en élastodiène, formant une âme élastique guipée ou recouverte d'un ou plusieurs fil(s) de couverture, des seconds fils de chaîne non élastiques (4), et des troisièmes fils de chaîne (5), lesdits premiers fils de chaîne (3) et lesdits seconds fils de chaîne (4) sont tissés selon une armure « pas de gaze », les seconds fils de chaîne (4) formant les fils de tour,
- les sens chaines et trames des bandes tissées sont parallèles entre-eux ou le sens chaîne ou trame d'une bande tissée donnée est disposé selon un angle alpha supérieur à 0° et inférieur à 90° par rapport au sens chaîne ou trame d'une autre bande tissée
- **en ce que** ledit panneau textile (26,27) est élastique dans la direction longitudinale (L) et présente dans la direction longitudinale (L) pour obtenir un allongement égal à 30%, une force qui est supérieure ou égale à 500 cN/cm,
- **en ce que** le nombre de fils de trame par centimètre dans chacune des bandes tissées est inférieure ou égale à 25,
- **en ce que** l'un au moins desdits fils de trame (2) comprend un nombre de filaments supérieur ou égal à 1 et inférieur ou égal à 5, chaque filament ayant un diamètre supérieur ou égal à 0,10 mm, et **en ce que** les seconds fils de chaîne (4) effectuent un tour pour une insertion d'un fil de trame (2).

2. Dispositif (20) selon la revendication **1, caractérisé en ce qu'**il comprend un dispositif de serrage démultiplicateur (30) de la force de pose manuelle dudit au moins un panneau textile (26,27), notamment un dispositif de serrage mécanique par câble(s) (30) et/ou par poulie(s).

3. Dispositif (20) selon l'une ou l'autre des revendications **1** et **2, caractérisé en ce que** l'un au moins desdits premiers fils de chaîne (3) est recouvert par au moins un premier fil de couverture.

4. Dispositif (20) selon l'une quelconque des revendications **1** à **3, caractérisé en ce que** l'un au moins desdits premiers fils de chaîne élastiques (3), éventuellement recouvert par au moins un premier fil de couverture, présente un allongement à rupture inférieur ou égal à 150%.

5. Dispositif (20) selon l'une quelconque des revendications **1** à 4, **caractérisé en ce que** l'un au moins desdits fils de trame (2) comprend un nombre de filaments supérieur ou égal à 1 et inférieur ou égal à 3, de préférence chaque filament ayant un diamètre supérieur ou égal à 0,1 mm.

6. Dispositif selon l'une quelconque des revendications **1** à 5, **caractérisé en ce que** la bande tissée (1) ou chacune desdites bandes tissées comprend une alternance de colonnes pleines (6) et de colonnes ajourées (7), chacune desdites colonnes pleines (6) et ajourées (7) ayant une direction longitudinale (l) correspondant au sens chaîne (C) de ladite bande tissée (1), les colonnes pleines (6) comprenant selon chacun de leurs bords longitudinaux (8) au moins un premier fil de chaîne (3) et un second fil de chaîne (4) assemblés selon une armure pas de gaze.

7. Dispositif selon la revendication 6, **caractérisé en ce que** les colonnes ajourées (7) sont constituées de fils de trame (2).

8. Dispositif (20) selon l'une quelconque des revendications **1** à 7, **caractérisé en ce que** ledit panneau textile (26,27) présente dans la direction longitudinale (L) pour obtenir un allongement égal à 30%, une force qui est supérieure ou égale à 700 cN/cm, de préférence supérieure ou égal à 1000 cN/cm et inférieure ou égale à 2500 cN/cm.

9. Procédé de fabrication d'un dispositif (20) selon l'une quelconque des revendications **1** à 8, **caractérisé en ce qu'**il comprend les étapes suivantes :
- une première étape de tissage de plusieurs bandes textiles tissées (28,29), chaque bande textile (1,28,29) étant tissée avec :
des premiers fils de chaîne élastiques (3) dont un ou des premier(s) fil(s) de chaine élastique(s) (3) est/sont un ou des fil(s) monofilamentaire(s), en élasthanne ou en élastodiène, formant une âme élastique guipée ou recouverte d'un ou plusieurs fil(s) de couverture,
des seconds fils de chaîne non élastiques (4), des troisièmes fils de chaîne (5), et des fils de trame (2),
les premiers fils de chaîne (3) et les seconds fils de chaîne (4) étant tissés selon une armure pas de gaze en sorte que les seconds fils de chaine (4) forment les fils de tour,
le nombre de fils de trame par centimètre dans chacune des bandes tissées est inférieure ou égale à 25, - l'un au moins desdits fils de trame (2) comprend un fil comprenant un nombre de filaments supérieur ou égal à 1 et inférieur ou égal à 5, chaque filament ayant un diamètre supérieur ou égal à 0,10 mm;
- une étape d'application d'une tension (daN) sur l'un au moins desdits premiers fils de chaîne élastiques (3) en sorte d'allonger ledit au moins premier fil de chaîne élastique (3) lors de ladite première étape, ladite étape d'application comprenant un nombre de tours des seconds fils de chaîne (4) égal à un pour une insertion d'un fil de trame (2);
- une étape de formation d'un panneau textile (26,27) ayant une direction longitudinale (L) et une direction transversale (T) comprenant plusieurs bandes textiles tissées superposées (28,29) en sorte que les sens chaines et trames des bandes tissées soient parallèles entre-eux ou en sorte que le sens chaîne ou trame d'une bande tissée donnée soit disposé selon un angle alpha supérieur à 0° et inférieur à 90° par rapport au sens chaîne ou trame d'une autre bande tissée, lequel panneau présente dans la direction longitudinale (L) pour obtenir un allongement égal à 30% une force qui est supérieure ou égale à 500 cN/cm.

10. Procédé de fabrication selon la revendication 9, **caractérisé en ce que** l'étape de mise en tension consiste à exercer une tension sur l'un au moins desdits premiers fils de chaîne élastiques (3) à l'aide d'un ou plusieurs dispositif(s) choisi(s) dans le groupe constitué par les dispositifs suivants: l'un au moins desdits premiers fils de chaîne élastiques (3) est recouvert par au moins un premier fil de couverture ayant un nombre de tours/mètre supérieur ou égal à 720 tours/m, et/ou une tension exercée sur l'un au moins desdits premiers fils de chaîne élastiques supérieure (3) ou égale à 10 cN.

## Patentansprüche

1. Medizinische Vorrichtung (20), die dazu vorgesehen ist, mindestens einen Bereich des Körpers zu unterstützen, insbesondere Knie- oder Sprunggelenkorthese, Stützgürtel (21) oder Stützband, umfassend mindestens eine Textilbahn (26, 27), die eine Längsrichtung (L) und eine Querrichtung (T) aufweist und derart eingerichtet ist, dass sie den zu unterstützenden Bereich des Körpers ganz oder teilweise umgibt, **dadurch gekennzeichnet, dass**:
- die Textilbahn mehrere gewebte Textilbänder (1, 28, 29) umfasst, die zumindest teilweise überlagert sind,
- die gewebten Bänder (1, 28, 29) jeweils eine Kettrichtung (C) und eine Schussrichtung (t) aufweisen und jeweils Schussfäden (2), erste elastische Kettfäden (3), von denen ein oder mehrere erste elastische Kettfäden (3) ein oder mehrere Monofilamentfäden aus Elasthan oder aus Elastodien ist/sind, die einen elastischen Kern ausbilden, der mit einem oder mehreren Deckfäden umwunden oder bedeckt ist, zweite nichtelastische Kettfäden (4) und dritte Kettfäden (5), wobei die ersten Kettfäden (3) und die zweiten Kettfäden (4) gemäß einer "Dreherbindung" gewebt sind, wobei die zweiten Kettfäden (4) Dreherfäden ausbilden, umfassen.
- die Kett- und die Schussrichtung der gewebten Bänder zueinander parallel sind oder die Kett- oder Schussrichtung eines gegebenen gewebten Bandes gemäß einem Winkel alpha von größer als 0° und kleiner als 90° in Bezug auf die Kett- oder Schussrichtung eines anderen gewebten Bandes angeordnet ist,
- dadurch, dass die Textilbahn (26, 27) in der Längsrichtung (L) elastisch ist und in der Längsrichtung (L), um eine Dehnung gleich 30 % zu erlangen, eine Kraft aufweist, die größer oder gleich 500 cN/cm ist,
- dadurch, dass die Anzahl von Schussfäden pro Zentimeter in jedem der gewebten Bänder kleiner oder gleich 25 ist,
- dadurch, dass mindestens einer der Schussfäden (2) eine Anzahl von Filamenten umfasst, die größer oder gleich 1 und kleiner oder gleich 5 ist, wobei jedes Filament einen Durchmesser größer oder gleich 0,10 mm aufweist,
und dadurch, dass die zweiten Kettfäden (4) eine Umschlingung zur Einführung eines Schussfadens (2) ausführen.

2. Vorrichtung (20) nach Anspruch 1, **dadurch gekennzeichnet, dass** sie eine Spannvorrichtung (30), welche die manuelle Anlegekraft der mindestens einen Textilbahn (26, 27) vervielfacht, insbesondere eine mechanische Spannvorrichtung mit Draht/Drähten (30) und/oder mit Rolle(n), umfasst.

3. Vorrichtung (20) nach einem der Ansprüche 1 und 2, **dadurch gekennzeichnet, dass** der mindestens eine der ersten Kettfäden (3) mit mindestens einem ersten Deckfaden bedeckt ist.

4. Vorrichtung (20) nach einem der Ansprüche 1 bis 3, **dadurch gekennzeichnet, dass** der mindestens eine der ersten elastischen Kettfäden (3), der gegebenenfalls mit mindestens einem ersten Deckfaden bedeckt ist, eine Bruchdehnung kleiner oder gleich 150 % aufweist.

5. Vorrichtung (20) nach einem der Ansprüche 1 bis 4, **dadurch gekennzeichnet, dass** der mindestens eine der Schussfäden (2) eine Anzahl von Filamenten von größer oder gleich 1 und kleiner oder gleich 3 umfasst, wobei jedes Filament bevorzugt einen Durchmesser größer oder gleich 0,1 mm aufweist.

6. Vorrichtung nach einem der Ansprüche 1 bis 5, **dadurch gekennzeichnet, dass** das gewebte Band (1) oder jedes der gewebten Bänder einen Wechsel von gefüllten Stegen (6) und durchbrochenen Stegen (7) umfasst, wobei jeder der gefüllten (6) und durchbrochenen (7) Stege eine Längsrichtung (I) aufweist, die der Kettrichtung (C) des gewebten Bandes (1) entspricht, wobei die gefüllten Stege (6) gemäß jeder ihrer Längskanten (8) mindestens einen ersten Kettfaden (3) und einen zweiten Kettfaden (4) umfassen, die gemäß einer Dreherbindung zusammengefügt sind.

7. Vorrichtung nach Anspruch 6, **dadurch gekennzeichnet, dass** die durchbrochenen Stege (7) aus Schussfäden (2) gebildet sind.

8. Vorrichtung (20) nach einem der Ansprüche 1 bis 7, **dadurch gekennzeichnet, dass** die Textilbahn (26, 27) in der Längsrichtung (L), um eine Dehnung gleich 30 % zu erlangen, eine Kraft aufweist, die größer oder gleich 700 cN/cm, bevorzugt größer oder gleich 1000 cN/cm, und kleiner oder gleich 2500 cN/cm ist.

9. Verfahren zur Herstellung einer Vorrichtung (20) nach einem der Ansprüche 1 bis 8, **dadurch gekennzeichnet, dass** es die folgenden Schritte umfasst:
- einen ersten Schritt des Webens mehrerer gewebter Textilbänder (28, 29), wobei jedes Textilband (1, 28, 29) gewebt wird mit:
ersten elastischen Kettfäden (3), von denen ein oder mehrere erste elastische Kettfäden (3) ein oder mehrere Monofilamentfäden aus Elasthan oder aus Elastodien ist/sind, die einen elastischen Kern ausbilden, der mit einem oder mehreren Deckfäden umwunden oder bedeckt ist,
zweiten nichtelastischen Kettfäden (4), dritten Kettfäden (5) und Schussfäden (2),
wobei die ersten Kettfäden (3) und die zweiten Kettfäden (4) gemäß einer Dreherbindung derart gewebt werden, sodass die zweiten Kettfäden (4) Dreherfäden ausbilden,
wobei die Anzahl von Schussfäden pro Zentimeter in jedem der gewebten Bänder kleiner oder gleich 25 ist,
- wobei der mindestens eine der Schussfäden (2) einen Faden umfasst, der eine Anzahl von Filamenten größer oder gleich 1 und kleiner oder gleich 5 umfasst, wobei jedes Filament einen Durchmesser größer oder gleich 0,10 mm aufweist,
- einen Schritt des Aufbringens einer Spannung (daN) auf den mindestens einen der ersten elastischen Kettfäden (3) derart, dass sich der mindestens erste elastische Kettfaden (3) während des ersten Schritts dehnt, wobei der Schritt des Aufbringens eine Anzahl von Umschlingungen der zweiten Kettfäden (4) gleich eins zur Einführung eines Schussfadens (2) umfasst,
- einen Schritt des Ausbildens einer Textilbahn (26, 27), die eine Längsrichtung (L) und eine Querrichtung (T) aufweist, umfassend mehrere überlagerte gewebte Textilbänder (28, 29) derart, dass die Kett- und die Schussrichtung der gewebten Bänder zueinander parallel sind, oder derart, dass die Kett- oder Schussrichtung eines gegebenen gewebten Bandes gemäß einem Winkel alpha von größer als 0° und kleiner als 90° in Bezug auf die Kett- oder Schussrichtung eines anderen gewebten Bandes angeordnet ist, wobei die Bahn in der Längsrichtung (L), um eine Dehnung gleich 30 % zu erlangen, eine Kraft aufweist, die größer oder gleich 500 cN/cm ist.

10. Verfahren zur Herstellung nach Anspruch 9, **dadurch gekennzeichnet, dass** der Schritt des Spannens darin besteht, eine Spannung auf den mindestens einen der ersten elastischen Kettfäden (3) mithilfe einer oder mehrerer Vorrichtungen auszuüben, die ausgewählt ist/sind aus der Gruppe bestehend aus den folgenden Vorrichtungen: der mindestens eine der ersten elastischen Kettfäden (3) wird mit mindestens einem ersten Deckfaden bedeckt, der eine Anzahl von Umschlingungen/Meter von größer oder gleich 720 Umschlingungen/m aufweist, und/oder einer auf den mindestens einen der ersten elastischen Kettfäden (3) ausgeübten Spannung größer oder gleich 10 cN.

## Claims

1. A medical device (20) for supporting at least one region of the body, especially knee or ankle orthesis, support belt (21) or support strip, comprising at least one textile panel (26,27) having a longitudinal direction (L) and a transversal direction (T) arranged so as to encircle all or part of said region of the body to be supported, **characterized in that**:
- said textile panel comprises several woven textile strips (1,28,29) at least partially superposed,
- said woven strips (1,28,29) each having a warp direction (C) and a weft direction (t), and each comprising weft threads (2), first elastic warp threads (3) of which one or several first elastic warp thread(s) (3) is/are monofilament thread(s), in elastane or in elastodiene, forming an covered or wrapped elastic core with one or several cover thread(s), second non-elastic warp threads (4), and third warp threads (5), said first warp threads (3) and said second warp threads (4) are woven according to a leno weave, the second warp threads (4) forming the doup threads,
- the warp and weft directions of woven strips are parallel to each other or the warp or weft direction of a given woven strip is arranged according to an alpha angle greater than 0° and lower than 90° relative to the warp or weft direction of another woven strip,
- **in that** said textile panel (26,27) is elastic in the longitudinal direction (L) and has a force in the longitudinal direction (L) which is greater than or equal to 500 cN/cm, to achieve an elongation equal to 30%,
- **in that** the number of weft threads per centimetre in each of the woven strips is less than or equal to 25,
- **in that** one at least of said weft threads (2) comprises a number of filaments greater than or equal to 1 and less than or equal to 5, each filament having a diameter greater than or equal to 0.10 mm,
and **in that** the second warp threads (4) make a turn for one insertion of a weft thread (2).

2. Device (20) according to claim 1, **characterized in that** it comprises a multiplier gripping device (30) of the manual application force of said at least one textile panel (26,27), especially a mechanical gripping device by cable(s) (30) and/or by pulley(s).

3. Device (20) according to one or other of claims 1 and 2, **characterized in that** one at least of said first warp threads (3) is covered by at least one first cover thread.

4. Device (20) according to any one of the claims 1 to 3, **characterized in that** one at least of said first elastic warp threads (3), optionally covered by at least one first cover thread, has breaking elongation less than or equal to 150%.

5. Device (20) according to any one of the claims 1 to 4, **characterized in that** one at least of said weft threads (2) comprises a number of filaments greater than or equal to 1 and less than or equal to 3, preferably each filament having a diameter higher or equal to 0.1 mm.

6. Device according to any one of the claims 1 to 5, **characterized in that** the woven strip (1) or each of said woven strips comprises alternating solid columns (6) and openwork columns (7), each of said solid (6) and openwork (7) columns having a longitudinal direction (l) corresponding to the warp direction (C) of said woven strip (1), the solid columns (6) comprising according to each of their longitudinal edges (8) at least one first warp thread (3) and a second warp thread (4) assembled according to a leno weave.

7. Device according to claim 6, **characterized in that** the openwork columns (7) are constituted by weft threads (2).

8. Device (20) according to any one of the claims 1 to 7, **characterized in that** to achieve elongation equal to 30%, said textile panel (26,27) has in the longitudinal direction (L) a force which is greater than or equal to 700 cN/cm, preferably the force is greater than or equal to 1000 cN/cm and less than or equal to 2500 cN/cm.

9. A manufacturing process of a device (20) according to any one of the claims 1 to 8, **characterized in that** it comprises the following steps:
- a first weaving step of several woven textile strips (28,29), each textile strip (1,28,29) being woven with:
first elastic warp threads (3) of which one or several first elastic warp thread(s) (3) is/are monofilament thread(s), in elastane or in elastodiene, forming a covered or wrapped elastic core with one or several cover thread(s),
second non-elastic warp threads (4), third warp threads (5), and weft threads (2),
the first warp threads (3) and the second warp threads (4) being woven according to a leno weave such that the second warp threads (4) form the doup threads,
the number of weft threads per centimetre in each of the woven strips is less than or equal to 25,
one at least of said weft threads (2) comprises a thread comprising a number of filaments greater than or equal to 1 and less than or equal to 5, each filament having a diameter greater than or equal to 0.10 mm;
a tension application step (daN) on one at least of said first elastic warp threads (3) so as to elongate said at least first elastic warp thread (3) during said first step, said application step comprising a number of turns of the second warp threads (4) equal to one for one insertion of a weft thread (2);
a shaping step of a textile panel (26,27) having a longitudinal direction (L) and a transversal direction (T) comprising several superposed woven textile strips (28,29) such that the warp and weft directions of woven strips are parallel to each other or the warp or weft direction of a given woven strip is arranged according to an alpha angle greater than 0° and lower than 90° relative to the warp or weft direction of another woven strip, which panel has in the longitudinal direction (L) a force which is greater than or equal to 500 cN/cm to achieve elongation equal to 30%.

10. The manufacturing process according to claim 9, wherein the tensioning step consists of exerting tension on one at least of said first elastic warp threads (3) by means of one or more device(s) selected in the group constituted by the following devices: one at least of said first elastic warp threads (3) is covered by at least one first cover thread having a number of turns/meter greater than or equal to 720 turns/meter, and/or tension exerted on one at least of said first elastic warp threads (3) greater than or equal to 10 cN.
